# EUROPÄISCHE PATENTSCHRIFT

(11) **EP 2 438 903 B1**
(45) Veröffentlichungstag und Bekanntmachung des Hinweises auf die Patenterteilung: **14.03.2018**
(21) Anmeldenummer: 11182471.0
(22) Anmeldetag: 23.09.2011
(51) Int. Cl.: A61K 8/35, A61K 8/40, A61K 8/49, A61Q 17/04

(54) **Konservierungsmittelfreies Sonnenschutzmittel**
Preservative-free sunscreen
Produit de protection solaire sans conservateur

(30) Priorität: 07.10.2010 DE 102010042147
(43) Veröffentlichungstag der Anmeldung: 11.04.2012
(73) Patentinhaber: Beiersdorf AG, 20253 Hamburg (DE)
(72) Erfinder: Zanforlin Trede, Lucia, 22769 Hamburg (DE); Schade, Juliane, 22529 Hamburg (DE)

(56) Entgegenhaltungen:
- EP-A2- 1 310 237
- WO-A1-2009/018975
- WO-A2-03/039506
- DE-A1- 10 155 963
- DE-A1-102007 024 342
- DE-A1-102007 024 343
- DE-A1-102008 018 786
- DE-A1-102008 018 787
- DE-A1-102008 033 343

## Beschreibung

Die vorliegende Erfindung betrifft ein konservierungsmittelfreies Sonnenschutzmittel.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen: Sonnenschutzmittel werden häufig beim Wassersport, beispielsweise beim Baden am Strand oder im Freibad, beim Surfen oder Segeln oder beim Schnorcheln, eingesetzt. In derartigen Anwendungsbereichen erwartet der Nutzer eine gewisse Wasserfestigkeit des Sonnenschutzmittels, damit die Haut auch nach dem Kontakt mit Wasser vor der UV-Strahlung geschützt bleibt und die Lichtschutzfilter nicht sofort von der Haut gewaschen werden.

Es war daher die Aufgabe der vorliegenden Erfindung, ein Sonnenschutzmittel mit höherer Wasserfestigkeit zu entwickeln.

Kosmetische Sonnenschutzmittel haben darüber hinaus den Nachteil, dass sie besonders stark konserviert werden müssen, um über einen längeren Zeitraum mikrobiologisch rein zu sein. Dieser Umstand ergibt sich aus den relativ langen "Aufbrauchzeiten" bereits geöffneter Sonnenschutzprodukte, der in der Regel relativ hohen Lagertemperatur (beispielsweise die direkte Lagerung in der Sonne am sommerlichen Strand), sowie dem Kontakt mit stark verkeimten Hautpartien (beispielsweise verschwitzten Händen). Andererseits stehen Konservierungsmittel (ob zu Recht oder zu Unrecht kann im Rahmen dieser Offenbarung dahingestellt bleiben) bei den Verbrauchern in dem Ruf, hinsichtlich ihrer Gesundheitsverträglichkeit und Umweltverträglichkeit nicht ganz unbedenklich zu sein.

Es war daher die Aufgabe der vorliegenden Erfindung, ein Sonnenschutzmittel zu entwickeln, dessen Gehalt an Konservierungsstoffen im Vergleich zum Stand der Technik deutlich reduziert ist.

Gelöst werden die Aufgaben durch eine kosmetische Zubereitung gemäß Anspruch 1.

Zwar sind dem Fachmann durchaus Sonnenschutzmittel aus dem Stand der Technik bekannt, welche eine UV-Filterkombination aus 4-(tert.-Butyl)-4'-methoxydibenzoylmethan, 2-Ethylhexyl-2-cyano-3,3-diphenylacrylat, 2-Phenylbenzimidazol-5-sulfonsäure und/oder deren Salze sowie 2,4-Bis-{[4-(2-ethyl-hexyloxy)-2-hydroxy]-phenyl}-6-(4-methoxyphenyl)-1,3,5-triazin enthalten, doch konnten diese Offenbarungen nicht den Weg zur vorliegenden Erfindung weisen, da diese Zubereitungen Konservierungsmittel "in der erforderlichen Menge (quantum satis, q.s.)" enthalten, wozu üblicherweise Parabene (Methylparaben, Ethylparaben, Propylparaben, Butylparaben) und Phenoxyethanol zählen.

Darüber hinaus kennt der Fachmann die Offenbarungen der US 2007/0190005, DE 10353030, DE 102004014616, DE 102008018787, DE 102008018786, EP 1310237, WO 03/039506, DE 10155963, DE 102007024342, DE 102007024342, WO 2009/018975 und DE 10200803343, die ebenfalls nicht den Weg zur vorliegenden Erfindung weisen konnten.

Erfindungsgemäß werden kosmetische Zubereitungen als "konservierungsmittelfrei" definiert, die weder Parabene (Methylparaben, Ethylparaben, Propylparaben, Butylparaben) noch Phenoxyethanol enthalten.

Es ist darüber hinaus erfindungsgemäß, wenn die Zubereitung frei ist von 3-lodo-2-propynylbutylcarbamat und/oder Methylisothiazolinon, die im Rahmen der vorliegenden Offenbarung ebenfalls zu den Konservierungsmitteln gezählt werden.

Erfindungsgemäß besonders bevorzugt sind die erfindungsgemäßen Zubereitungen daher konservierungsmittelfrei.

Erfindungsgemäß bevorzugte Ausführungsformen der vorliegenden Erfindung sind dadurch gekennzeichnet, dass die Zubereitung von 6 bis 10 Gew.-% Ethanol, bezogen auf das Gesamtgewicht der Zubereitung, enthält.

Erfindungsgemäß wird Ethanol nicht zu den Konservierungsmitteln gezählt.

Es ist erfindungsgemäß vorteilhaft, wenn die Zubereitung 4-(tert.-Butyl)-4'-methoxydibenzoylmethan in einer Gesamtkonzentration von 0,1 bis 10 Gew.-%, bezogen auf das Gesamtgewicht der Zubereitung, enthält.

Es ist erfindungsgemäß bevorzugt, wenn die Zubereitung 4-(tert.-Butyl)-4'-methoxydibenzoylmethan in einer Gesamtkonzentration von 3 bis 5 Gew.-%, bezogen auf das Gesamtgewicht der Zubereitung, enthält.

Erfindungsgemäß vorteilhafte Ausführungsformen der vorliegenden Erfindung sind dadurch gekennzeichnet, dass die Zubereitung 2-Phenylbenzimidazol-5-sulfonsäure und/oder deren Salze in einer Gesamtkonzentration von 0,1 bis 5 Gew.-%, bezogen auf das Gesamtgewicht der Zubereitung, enthält.

Erfindungsgemäß bevorzugte Ausführungsformen der vorliegenden Erfindung sind dadurch gekennzeichnet, dass die Zubereitung 2-Phenylbenzimidazol-5-sulfonsäure und/oder deren Salze in einer Gesamtkonzentration von 1 bis 3 Gew.-%, bezogen auf das Gesamtgewicht der Zubereitung, enthält.

Als erfindungsgemäß vorteilhafte Salze werden dabei die Natrium-, Kalium-. Ammonium-, Triethanolammonium- und andere Alkylammonium- und Hydroxyalkylammoniumsalze der 2-Phenylbenzimidazol-5-sulfonsäure angesehen.

Es ist erfindungsgemäß vorteilhaft, wenn die Zubereitung 2,4-Bis-{[4-(2-ethyl-hexyloxy)-2-hydroxy]-phenyl}-6-(4-methoxyphenyl)-1,3,5-triazin in einer Gesamtkonzentration von 0,1 bis 5 Gew.-%, bezogen auf das Gesamtgewicht der Zubereitung, enthält.

Es ist erfindungsgemäß bevorzugt, wenn die Zubereitung 2,4-Bis-{[4-(2-ethyl-hexyloxy)-2-hydroxy]-phenyl}-6-(4-methoxyphenyl)-1,3,5-triazin in einer Gesamtkonzentration von 0,5 bis 3,5 Gew.-%, bezogen auf das Gesamtgewicht der Zubereitung, enthält.

Darüber hinaus sind erfindungsgemäß vorteilhafte Ausführungsformen der vorliegenden Erfindung dadurch gekennzeichnet, dass die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titiandioxid; Zinkoxid in einer Konzentration von 0,01 bis 40 Gewichts-% bezogen auf das Gesamtgewicht der Zubereitung.

Erfindungsgemäß vorteilhaft ist die erfindungsgemäße Zubereitung frei von p-Methylbenzylidencampher. Generell ist es von Vorteil, Zubereitungen ohne UV-Filter auf Campher-Basis herzustellen.

Darüber hinaus ist es erfindungsgemäß vorteilhaft, wenn die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, β-Alanin, Tocopherylacetat, Harnstoff; Hyaluronsäure; Dihydroxyaceton; 8-Hexadecen-1,16-dicarbonsäure, Glycyrrhetinsäure, Glucosylglyceride und/oder Licochalcon A enthält.

Erfindungsgemäß liegt die erfindungsgemäße Zubereitung in Form einer Öl-in-Wasser-Emulsion (O/W-Emulsion) vor. Es ist erfindungsgemäß vorteilhaft, wenn die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Poiyglyceryl-3-methylglycosedistearat, Ceteareth-20, PEG-40 Stearat und Natriumcetearylsulfat enthält.

Erfindungsgemäß besonders bevorzugt ist dabei der Einsatz von Ceteareth-20 oder Glycerylstearatcitrat.

Diese erfindungsgemäßen O/W-Emulgatoren können erfindungsgemäß vorteilhaft in einer Konzentration von 0,1 bis 10 Gewichts-% und bevorzugt in einer Konzentration von 0,2 bis 7 Gewichts-%, bezogen auf das Gesamtgewicht der Zubereitung in dieser enthalten sein.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr, 7631-86-9), Talkum, Lauroyl Lysine und Acrylonitrile-methacrylonitrile-methyl-methacrylate.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl wie Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Schaumstabilisatoren, Elektrolyte, etc.. Diese Verbindungen werden erfindungsgemäß nicht zu den Konservierungsmitteln gezählt.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*)*.*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSi0_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Die erfindungsgemäßen Zubereitungen werden erfindungsgemäß bevorzugt als Sonnenschutzmittel verwendet. Darüber hinaus ist ihr Einsatz als Tagespflegeprodukt (insbesondere als Gesichtspflegeprodukt oder zur Handpflege) erfindungsgemäß.

Erfindungsgemäß ist nicht zuletzt die Verwendung einer Kombination aus
a) 4-(tert.-Butyl)-4'-methoxydibenzoylmethan,
b) 2-Ethylhexyl-2-cyano-3,3-diphenylacrylat,
c) 2-Phenylbenzimidazol-5-sulfonsäure und/oder deren Salze,
d) 2,4-Bis-{[4-(2-ethyl-hexyloxy)-2-hydroxy]-phenyl}-6-(4-methoxyphenyl)-1,3,5-triazin und Ethanol zur Herstellung wasserfester und/oder konservierungsmittelfreier kosmetischer Zubereitungen, insbesondere Sonnenschutzmittel, wobei unter konservierungsmittelfrei Zubereitungen verstanden werden, die keine Stoffe enthalten, die im Rahmen dieser Offenbarung als Konservierungsmittel gekennzeichnet sind.

### Vergleichsversuche

Mit dem folgenden Vergleichsversuch konnte der erfindungsgemäße Effekt belegt werden: Es wurden die folgenden Rezepturen hergestellt und deren Wasserfestigkeit gemäß den im Dezember 2005 von COLIPA veröffentlichten Leitlinien "Guidelines for Evaluating Sun Product Water Resistance" bestimmt.

Der an den Probanden vor und nach dem Eintauchen in Wasser bestimmte Lichtschutzfaktor (LSF) eines Sonnenschutzmittels ist definiert als das Verhältnis der minimalen Erythemdosis (MED) auf der geschützten Haut zur MED auf der ungeschützten Haut des gleichen Probanden. LSF und MED wurden nach der "Internationalen Methode zur Bestimmung des Lichtschutzfaktors (LSF)" bestimmt. Alle Bestimmungen des LSF vor und nach dem Eintauchen in Wasser wurden im gleichen Labor mit den gleichen Probanden in der gleichen Testsequenz durchgeführt.

**Rezepturen**

| | **Produkt 1** | **Produkt 2** |
|---|---|---|
| **INCI-Name(n)** | **m [%]** | **m [%]** |
| Octocrylene | 8.00 | 8.00 |
| Bis-Ethylhexyloxyphenol Methoxyphenyl Triazine | 3.50 | 3.50 |
| Butyl Methoxydibenzoylmethane | 4.50 | 4.50 |
| Phenylbenzimidazole Sulfonic Acid | 1.00 | 1.00 |
| Weitere UV-Filter (Ethylhexyl Methoxycinnamate, Diethylhexyl Butamido Triazone, Homosalate und Titanium Dioxide, in beiden Rezepturen in der identischen Zusammensetzung) | 5.00 | 5.00 |
| Komplexbildner | 0.20 | 0.20 |
| Verdicker | 0.40 | 0.40 |
| Moisturizer | 10.00 | 10.0 |
| Neutralisationsmittel | 0.33 | 0.33 |
| Filmbildner | 0.50 | 0.50 |
| Emulgator | 1.00 | 1.00 |
| Öle | 6.50 | 6.50 |
| Wachse | 1.00 | 1.00 |
| Alcohol Denat. | 6.00 | 8.00 |
| Phenoxyethanol | 0.60 | - |
| Ethylparaben | 0.10 | - |
| Methylparaben | 0.30 | - |
| Aqua | Ad 100,0 | Ad 100,0 |

**Wasserfestigkeitsergebnisse:**

| | **Mean Value** | | | |
|---|---|---|---|---|
| | **LSF** | | | |
| | **static** | **wet** | **Water Resistence [%]** | **95 % confidence interval (CI) [%]** |
| Produkt 1 | 55,6 | 25,2 | 44,8 | 16 |
| Produkt 2 | 86,1 | 50,2 | 58,8 | 12,5 |

Fazit: Die Formulierung welche frei ist von Parabenen und Phenoxyethanol zeigt eine höhere Wasserfestigkeit als jene Rezeptur, die das gleiche UV-Filter-System aus Octocrylene, Bis-Ethylhexyloxyphenol Methoxyphenyl Triazine, Butyl Methoxydibenzoylmethane und Phenylbenzimidazole Sulfonic Acid enthält und mit Parabenen sowie Phenoxyethanol versetzt ist.

Mit dem Produkt 1 wurde keine lang anhaltende Wasserfestigkeit bestätigt. Der Wert liegt bei 44,8 %, d. h. unter 50 %. Das Produkt 2 weist eine lang anhaltende Wasserfestigkeit auf, da der SPF-Wert nach dem Wässern 58,8 % des SPF-Werts vor dem Wässern entspricht.

### Beispiele

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

| | **1** | **2** | **3** | **4** | **5** | **6** | **7** | **8** | **9** | **10** | **12** |
|---|---|---|---|---|---|---|---|---|---|---|---|
| Acrylat/C10-30 Alkyl Acrylat Crosspolymer | | 0,2 | | 0,2 | | | | | 0,2 | 0,2 | 0,4 |
| Carbomer | | | | 0,2 | | | | | | | 0,2 |
| Xanthan Gummi | 0,2 | 0,3 | | | | | | | | 0,2 | |
| Copolymer aus Vinylpyrrolidon und Acrylsäure | | | 0,4 | | | 0,3 | | | | | 0,2 |
| Tapioca Stärke | 1 | | | | | | | | | | |
| Distarch Phosphate | | | 1 | | | | | | | | |
| Carrageenan | | 0,2 | | | | | | | 0,25 | | |
| VP/Hexadecene Copolymer | 1,5 | 1 | | 0,5 | 0,5 | 0,5 | 1 | 0,75 | 1 | 0,5 | 0,5 |
| Acrylates/C12-22 Alkylmethacrylate Copolymer | | | 1,5 | | | | | 1 | 2 | | |
| C18-36 Acid Triglyceride | | | 1 | | | 1 | | 0,5 | | 1 | |
| Natrium Stearoylglutamat | | 0,1 | 0,2 | 0,2 | | | | | | | |
| Sucrosepolystearat | | 0,8 | 1 | | | | | | | | |
| Sodium Cetearyl Sulfate | 1,5 | | | | | | | | | | |
| Cetearyl Alkohol + PEG-40 Castor Oil + Natrium Cetearyl Sulfat | | | | | | 2 | | 2,5 | | | |
| Glyceryl Stearat SE | 0,7 | | | | 1 | 1 | | 1 | | | |
| Cetearyl Alcohol 90 + Sodium Cetearyl Sulfate 10 | | | | | 1,5 | | | | | | |
| Cetyl PEG/PPG-10/1 Dimethicone | | | | | | | 1 | | | | |
| Glyceryl Stearate Citrate | | | | | | | | | | 2 | |
| Ceteareth-20 | | | | | | | | | 1 | | |
| Dibutyl Adipate | | 3 | | | | 2 | | | | 2 | 1 |
| Myristyl Myristat | 1 | 1 | | | | 0,5 | | 0,5 | | | |
| Butylenglycol Dicaprylat/Dicaprat | 1,5 | | 3 | | | | 3 | | 3 | | |
| C12-15 Alkyl Benzoat | 5 | | | 2 | 3 | 2 | 2 | | | | 2,5 |
| Caprylic/Capric Triglycerid | | | | 2 | | | | | | 3 | |
| Cetearylalkohol | | | | 1 | 1,5 | 0,5 | | 0,5 | | | |
| Cetyl Alkohol | 2 | | | | 2 | | | 1 | | | |
| Cyclomethicon | | | 5 | | | | | | | 1 | |
| Dicaprylylcarbonat | | 3 | 3 | | 3 | | | | 2 | | |
| Dimethicon | | | | | | 1 | 5 | | | | |
| Octyldodekanol | 4 | 5,5 | 3 | | 3,5 | | 4 | 3,5 | 6 | 2 | |
| Coco-Caprylate | | | | | | 3 | | | | 1 | |
| Isopropyl Palmitate | 1 | | | | | | 2 | | | 2 | 2 |
| Bis-Ethylhexyloxyphenol Methoxyphenyl Triazin | 2 | 1 | 1,5 | 2,5 | 0,5 | 3 | 1,5 | 1 | 2 | 2 | 3 |
| Butyl Methoxydibenzoylmethan | 4,5 | 3 | 3,5 | 4 | 4,5 | 4 | 4 | 3 | 4 | 5 | 4.75 |
| Diethylamino Hydroxybenzoyl Hexyl Benzoat | | 2 | | | | | | 1 | | | |
| Ethylhexyl Methoxycinnamat | | | | | | | | | 0,5 | 5 | 0,5 |
| Ethylhexyltriazone | | | | 1 | 0,5 | | | | | | |
| Homosalat | | 4 | | 5 | | | 2 | | | 2 | |
| Octocrylen | 5 | 6 | 8 | 6,5 | 7,5 | 6 | 9 | 7 | 6 | 7 | 8,5 |
| Octylsalicylat | | | | 3 | | 4 | 2 | 1 | | | |
| Polysllicon-15 | | 2 | | | | 3 | | | | | |
| Phenylbenzimidazol Sulfonsäure | 1 | 2 | 1,5 | 3 | 2,5 | 2 | 2 | 1 | 1,5 | 3,5 | 2 |
| Titandioxid | 2 | | 4 | 2 | 1,5 | | | | 2 | | 3 |
| Tris-(biphenyl)-1,3,5 triazin | | | 2 | | | | | | 1 | | |
| Tocopherol Acetate | 0,5 | 1 | 0,5 | 0,5 | 1 | 0,5 | 1 | 0,5 | 1 | 0,5 | 2 |
| Glycerin | 5 | 6 | 10 | 9 | 8 | 7 | 7 | 9 | 10 | 12 | 9 |
| Glycyrrhetinsäure | 0,1 | | | | | | | | 0,2 | | |
| Ubiquinone | | 0,3 | | | | | | | | | 0,5 |
| Alcohol Denat. | 6 | 8 | 7 | 9 | 10 | 7 | 8 | 8 | 6 | 7 | 6,5 |
| EDTA | 1 | 1 | 1 | 1 | 1 | 1 | 1 | 1 | 1 | 1 | 1 |
| Neutralisationsmittel (z. B. Sodium Hydroxide) | q.s. | q.s. | q.s. | q.s. | q.s. | q.s | q.s | q.s | q.s | q.s | q.s |
| Wasser | ad 100 | ad 100 | ad 100 | ad 100 | ad 100 | ad 100 | ad 100 | ad 100 | ad 100 | ad 100 | ad 100 |
| | | | | | | | | | | | |
| pH-Wert | 7,5 | 7,5 | 7,5 | 7,5 | 7,5 | 7,5 | 7,5 | 7,5 | 7,5 | 7,5 | 7,5 |

## Patentansprüche

1. Kosmetische Zubereitung in Form einer Öl-in-Wasser-Emulsion (O/W-Emulsion), enthaltend eine UV-Filterkombination aus
a) 4-(tert.-Butyl)-4'-methoxydibenzoylmethan,
b) 5 bis 9 Gew.-% bezogen auf das Gesamtgewicht der Zubereitung an 2-Ethylhexyl-2-cyano-3,3-diphenylacrylat,
c) 2-Phenylbenzimidazol-5-sulfonsäure und/oder deren Salze,
d) 2,4-Bis-{[4-(2-ethyl-hexyloxy)-2-hydroxy]-phenyl}-6-(4-methoxyphenyl)-1,3,5-triazin, wobei die Zubereitung frei ist von Parabenen, Phenoxyethanol, 3-lodo-2-propynylbutylcarbamat und Methylisothiazolinon, **dadurch gekennzeichnet, dass** die Zubereitung von 2 bis 12 Gew.-% Ethanol, bezogen auf das Gesamtgewicht der Zubereitung, enthält.

2. Kosmetische Zubereitung nach einem der vorhergehenden Ansprüche, **dadurch gekennzeichnet, dass** die Zubereitung 4-(tert.-Butyl)-4'-methoxydibenzoylmethan in einer Gesamtkonzentration von 0,1 bis 10 Gew.-%, bezogen auf das Gesamtgewicht der Zubereitung, enthält.

3. Kosmetische Zubereitung nach einem der vorhergehenden Ansprüche, **dadurch gekennzeichnet, dass** die Zubereitung 2-Phenylbenzimidazol-5-sulfonsäure und/oder deren Salze in einer Gesamtkonzentration von 0,1 bis 5 Gew.-%, bezogen auf das Gesamtgewicht der Zubereitung, enthält.

4. Kosmetische Zubereitung nach einem der vorhergehenden Ansprüche, **dadurch gekennzeichnet, dass** die Zubereitung 2,4-Bis-{[4-(2-ethyl-hexyloxy)-2-hydroxy]-phenyl}-6-(4-methoxyphenyl)-1,3,5-triazin in einer Gesamtkonzentration von 0,1 bis 5 Gew.-%, bezogen auf das Gesamtgewicht der Zubereitung, enthält.

5. Kosmetische Zubereitung nach einem der vorhergehenden Ansprüche, **dadurch gekennzeichnet, dass** die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)-ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titiandioxid; Zinkoxid in einer Konzentration von 0,01 bis 40 Gewichts-% bezogen auf das Gesamtgewicht der Zubereitung.

6. Kosmetische Zubereitung nach einem der vorhergehenden Ansprüche, **dadurch gekennzeichnet, dass** die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, β-Alanin, Tocopherylacetat, Harnstoff; Hyaluronsäure; Dihydroxyaceton; 8-Hexadecen-1,16-dicarbonsäure, Glycyrrhetinsäure, Glucosylglyceride und/oder Licochalcon A enthält.

## Claims

1. Cosmetic preparation in the form of an oil-in-water emulsion (O/W emulsion), comprising a UV filter combination of
a) 4-(tert-butyl)-4'-methoxydibenzoylmethane,
b) 5 to 9 wt% 2-ethylhexyl 2-cyano-3,3-diphenylacrylate, based on the total weight of the preparation,
c) 2-phenylbenzimidazole-5-sulfonic acid and/or salts thereof,
d) 2,4-bis{[4-(2-ethylhexyloxy)-2-hydroxy]phenyl}-6-(4-methoxyphenyl)-1,3,5-triazine,
wherein the preparation is free of parabens, phenoxyethanol, 3-iodo-2-propynyl butylcarbamate and methylisothiazolinone,
**characterized in that** the preparation comprises 2 to 12 wt% ethanol, based on the total weight of the preparation.

2. Cosmetic preparation according to one of the preceding claims, **characterized in that** the preparation comprises 4-(tert-butyl)-4'-methoxydibenzoylmethane at a total concentration of 0.1 to 10 wt%, based on the total weight of the preparation.

3. Cosmetic preparation according to either of the preceding claims, **characterized in that** the preparation comprises 2-phenylbenzimidazole-5-sulfonic acid and/or salts thereof at a total concentration of 0.1 to 5 wt%, based on the total weight of the preparation.

4. Cosmetic preparation according to any of the preceding claims, **characterized in that** the preparation comprises 2,4-bis{[4-(2-ethylhexyloxy)-2-hydroxy]phenyl}-6-(4-methoxyphenyl)-1,3,5-triazine at a total concentration of 0.1 to 5 wt%, based on the total weight of the preparation.

5. Cosmetic preparation according to any of the preceding claims, **characterized in that** the preparation comprises one or more further UV filters selected from the group of compounds comprising phenylene-1,4-bis(2-benzimidazyl)-3,3'-5,5'-tetrasulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulphonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane / dimethylsiloxane - copolymer; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine; merocyanine; titanium dioxide; zinc oxide at a concentration of 0.01 to 40 wt% based on the total weight of the preparation.

6. Cosmetic preparation according to any of the preceding claims, **characterized in that** the preparation comprises as further ingredients one or more compounds selected from the group of compounds comprising alpha-lipoic acid, folic acid, phytoene, D-biotin, coenzyme Q10, alpha-glucosylrutin, carnitine, carnosine, polidocanol, natural and/or synthetic isoflavonoids, flavonoids, creatine, creatinine, taurine, β-alanine, tocopheryl acetate, urea; hyaluronic acid; dihydroxyacetone; 8-hexadecene-1,16-dicarboxylic acid, glycyrrhetic acid, glucosyl glycerides and/or licochalcone A.

## Revendications

1. Préparation cosmétique sous la forme d'une émulsion huile dans eau (émulsion H/E), contenant une combinaison de filtres UV constituée par
a) le 4-(tert.-butyl)-4'-méthoxydibenzoylméthane,
b) 5 à 9 % en poids, par rapport au poids total de la préparation, de 2-cyano-3,3-diphénylacrylate de 2-éthylhexyle,
c) l'acide 2-phénylbenzimidazole-5-sulfonique et/ou ses sels,
d) la 2,4-bis-{[4-(2-éthyl-hexyloxy)-2-hydroxy]-phényl}-6-(4-méthoxyphényl)-1,3,5-triazine,
la préparation étant exempte de parabènes, de phénoxyéthanol, de carbamate de 3-iodo-2-propynylbutyle et de méthylisothiazolinone, **caractérisée en ce que** la préparation contient 2 à 12 % en poids d'éthanol, par rapport au poids total de la préparation.

2. Préparation cosmétique selon l'une quelconque des revendications précédentes, **caractérisée en ce que** la préparation contient du 4-(tert.-butyl)-4'-méthoxydibenzoylméthane en une concentration totale de 0,1 à 10 % en poids, par rapport au poids total de la préparation.

3. Préparation cosmétique selon l'une quelconque des revendications précédentes, **caractérisée en ce que** la préparation contient de l'acide 2-phénylbenzimidazole-5-sulfonique et/ou ses sels en une concentration totale de 0,1 à 5 % en poids, par rapport au poids total de la préparation.

4. Préparation cosmétique selon l'une quelconque des revendications précédentes, **caractérisée en ce que** la préparation contient de la 2,4-bis-{[4-(2-éthyl-hexyloxy)-2-hydroxy]-phényl}-6-(4-méthoxyphényl)-1,3,5-triazine en une concentration totale de 0,1 à 5 % en poids, par rapport au poids total de la préparation.

5. Préparation cosmétique selon l'une quelconque des revendications précédentes, **caractérisée en ce que** la préparation contient un ou plusieurs filtres UV supplémentaires, choisis dans le groupe des composés constitué par les sels de l'acide phénylène-1,4-bis-(2-benzimidazyl)-3,3'-5,5'-tétrasulfonique ; le 1,4-di(2-oxo-10-sulfo-3-bornylidène-méthyl)-benzène et ses sels ; les sels de l'acide 4-(2-oxo-3-bornylidène-méthyl)benzène-sulfonique ; les sels de l'acide 2-méthyl-5-(2-oxo-3-bornylidène-méthyl)sulfonique ; le 2,2'-méthylène-bis-(6-(2H-benzotriazol-2-yl)-4-(1,1,3,3-tétraméthylbutyl)-phénol) ; le 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; le 3-(4-méthylbenzylidène)camphre ; le 3-benzylidène-camphre ; le salicylate d'éthylhexyle ; l'acide téréphtalidène-dicamphre-sulfonique ; l'ester 2-éthylhexylique de l'acide 4-(diméthylamino)-benzoïque ; l'ester amylique de l'acide 4-(diméthylamino)benzoïque ; l'ester di(2-éthylhexylique) de l'acide 4-méthoxybenzalmalonique ; l'ester 2-éthylhexylique de l'acide 4-méthoxycinnamique ; l'ester isoamylique de l'acide 4-méthoxycinnamique ; la 2-hydroxy-4-méthoxybenzophénone, la 2-hydroxy-4-méthoxy-4'-méthylbenzophénone ; la 2,2'-dihydroxy-4-méthoxybenzophénone ; l'ester hexylique de l'acide 2-(4'-diéthylamino-2'-hydroxybenzoyl)-benzoïque ; le salicylate d'homomenthyle ; le 2-hydroxybenzoate de 2-éthylhexyle ; le benzalmalonate de diméthicodiéthyle ; le copolymère de 3-(4-(2,2-bis-éthoxycarbonylvinyl)-phénoxy)propényl)-méthoxysiloxane/diméthylsiloxane ; la dioctylbutylamidotriazone (INCI : Diethylhexyl-Butamidotriazone) ; la 2,4-bis-[5-1(diméthyl-propyl)benzoxazol-2-yl-(4-phényl)-imino]-6-(2-éthylhexyl)-imino-1,3,5-triazine de n° CAS 288254-16-0) ; l'ester tris-(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; la 2,4,6-tribiphényl-4-yl-1,3,5-triazine ; la mérocyanine ; le dioxyde de titane ; l'oxyde de zinc, en une concentration de 0,01 à 40 % en poids par rapport au poids total de la préparation.

6. Préparation cosmétique selon l'une quelconque des revendications précédentes, **caractérisée en ce que** la préparation contient en tant que constituants supplémentaires un ou plusieurs composés choisis dans le groupe de composés constitué par l'acide alpha-lipoïque, l'acide folique, le phytoène, la D-biotine, la coenzyme Q10, l'alpha-glucosylrutine, la carnitine, la carnosine, le polydocanol, les isoflavonoïdes naturels et/ou synthétiques, les flavonoïdes, la créatine, la créatinine, la taurine, la β-alanine, l'acétate de tocophéryle, l'urée ; l'acide hyaluronique ; la dihydroxyacétone; l'acide 8-hexadécène-1,16-dicarboxylique, l'acide glycyrrhétique, les glucosylglycérides et/ou la licochalcone A.
